# EUROPÄISCHE PATENTSCHRIFT

(11) **EP 3 104 157 B1**
(45) Veröffentlichungstag und Bekanntmachung des Hinweises auf die Patenterteilung: **03.01.2018**
(21) Anmeldenummer: 16171611.3
(22) Anmeldetag: 27.05.2016
(51) Int. Cl.: G01N 3/08, G01N 33/00

(54) **PRÜFVORRICHTUNG FÜR TABLETTEN**
TESTING DEVICE FOR TABLETS
DISPOSITIF DE VÉRIFICATION DE COMPRIMÉS

(30) Priorität: 12.06.2015 DE 102015109377
(43) Veröffentlichungstag der Anmeldung: 14.12.2016
(73) Patentinhaber: Kraemer, Thilo, 64291 Darmstadt (DE)
(72) Erfinder: Kraemer, Thilo, 64291 Darmstadt (DE)
(74) Vertreter: Hamel, Armin

(56) Entgegenhaltungen:
- DE-A1- 4 241 985
- DE-A1- 19 654 612

## Beschreibung

Gegenstand der vorliegenden Erfindung ist eine Vorrichtung zur Prüfung von Tabletten. Im Rahmen der Qualitätskontrolle von Tabletten ist es notwendig, diese auf ihre Eigenschaften wie Länge, Breite, Bruchfestigkeit und Gewicht zu überprüfen. Das Deutsche Arzneibuch enthält die hierfür relevanten gesetzlichen Bestimmungen. Dieser Vorgang wird teilweise in Prüfstationen durchgeführt, in die die Tabletten oder Oblongs manuell eingelegt werden. Bei größeren zu prüfenden Stückzahlen sollte nach Möglichkeit die Prüfung automatisch vonstattengehen, sodass eine Vielzahl von Tabletten innerhalb kurzer Zeit überprüft werden kann. Zudem muss gewährleistet werden, dass Tabletten unterschiedlicher Formen und Größen richtig positioniert werden können, um eine fehlerfreie Messung zu ermöglichen.

Auch bei der Herstellung von Tablets in der Waschmittelindustrie, werden mechanisch-physikalische Eigenschaften derselben, wie z. B. Gewicht, Abmessungen, Zerfallszeit in einem Medium und Härte bestimmt.

Der Stand der Technik kennt mehrere Vorrichtungen, die hierfür geeignet sind.

Beispielsweise sind Tablettenprüfsysteme bekannt, welche jeweils eine Mehrzahl von Tabletten aus einem Produktionszyklus auf diese Eigenschaften hin untersuchen. Tabletten einer Charge werden aus einem Vorratsbehälter vereinzelt und z. B. mittels eines Transportsterns oder mittels eines Transportbandes von einer Messstation zur nächsten befördert. Derartige Transportsterne besitzen peripher in der Regel 24 Kammern zur vereinzelten Aufnahme je eines Tablettenprüflings. Zuerst erfolgt ein Wiegevorgang, an den sich eine Messeinrichtung zur Ermittlungen der Abmessungen des Prüflings anschließt, auf die ein Härtetester folgt. Das Zusammenspiel der einzelnen Stationen sowie die Speicherung und Übermittlung der Messergebnisse übernimmt eine Software sowie eine zentrale Recheneinheit.

Die Härte eines Prüflings wird üblicherweise in einer fein auflösenden Kraftmessdose gemessen, die einen Druckkolben und ein Gegenlager, nämlich einen feststehenden Gegenbacken und beweglichen Pressbacken, aufweist. Der Prüfling wird in den Bereich zwischen Gegenbacken und Pressbacken auf eine Führungsbahn befördert, wobei der Prüfling vorzugsweise den Gegenbacken berührt. Der Pressbacken wird nun mittels eines Schrittmotors gegen den Gegenbacken und den vor diesem liegenden Prüfling gefahren. Die vom Pressbacken mit jedem Schritt des Motors ausgeübte Kraft wird gemessen und aufgezeichnet, die konstant und sehr klein ist, solange der Gegenbacken den Prüfling nicht berührt oder dieser ohne den Gegendruck des Gegenbackens über die Führungsbahn geschoben wird. Wenn der Pressbacken den Prüfling gegen den Gegenbacken drückt, steigt die von ihm ausgeübte Kraft mit jedem Schritt des Schrittmotors so lange an, bis der Prüfling zerbricht. Die dazu aufgewendete Kraft wird aufgezeichnet und dient als Maß für die Härte des Prüflings. Das plötzliche Abfallen der vom Pressbacken aufgewendeten Kraft beim Zerbrechen des Prüflings dient als Abbruchbedingung für das Beenden der Messung. Der Pressbacken wird in seine Ausgangsposition zurückgefahren, und der nächste Prüfling kann geprüft werden.

Eine solche Vorrichtung zur Durchführung eines Härtetests ist durch die WO 98/53298 A1 bekannt geworden, die einen Prüftisch zur Aufnahme des Prüflings sowie einen linear verfahrbaren Druckkolben und ein Gegenlager aufweist, die oberhalb des Prüftisches angeordnet und gegeneinander verfahrbar sind. Der Prüfling befindet sich zwischen Druckkolben und Gegenlager, wobei die beim Gegeneinanderdrücken der beiden aufgewendete Kraft oder eine dazu proportionale Größe mittels einer Kraftmessvorrichtung messbar und die Härte des Prüflings daraus bestimmbar wird.

Ein Prüfling muss zur Bestimmung seines Durchmessers wie auch zur Durchführung eines Härtetests waagrecht auf der Führungsbahn aufliegen, denn nur die in dieser Lage gemessene Kraft beim Härtetest ist aussagekräftig und zum Beispiel pharmazeutisch zugelassen.

Die Gebrauchsmusterschrift DE 298 24 199 U1 offenbart ein System zur Durchführung von Härtetests an Prüfkörpern, bei der die Tablette auf einem Prüftisch platziert wird. Es wird aber keine Ausrichtung der Tablette durchgeführt. Stattdessen wird durch eine Vorrichtung zur Lageerkennung die Ausrichtung des Prüfkörpers festgestellt. Daraufhin werden eine bewegliche Backe sowie eine feste Backe, die zum Härtetest der Tablette dienen, entsprechend ausgerichtet, damit die Überprüfung der Tablette erfolgen kann. Diese Erfindung setzt jedoch einen komplizierten und kostspieligen Aufbau voraus, der Mittel zur Bilderkennung umfassen muss.

Die PCT-Veröffentlichungsschrift WO 2011/035818 A1 zeigt ein ähnliches System, bei dem die Lage eines Prüfkörpers zunächst mittels eines optischen Systems erkannt wird. Die Ausrichtung des Prüfkörpers kann hierbei aber auch dadurch erfolgen, dass sich die Unterlage, auf der sich die Tablette befindet, dreht. Auch hier stellt sich das Problem, dass ein vergleichsweise komplizierter Aufbau notwendig ist, um die Ausrichtung der Tablette festzustellen.

Ein alternativer Ansatz wird in den Druckschriften DE 10 2007 054 908 B4 und DE 10 2007 054 909 B4 verfolgt, wobei der Prüfkörper durch Ausrichtzangen ausgerichtet wird und mittels eines Schiebers zwischen den Backen positioniert und ggf. ausgerichtet wird. Der Prüfling wird mittels eines rotierenden Transportsterns gefördert.

In der Druckschrift DE 42 41 985 A1 wird eine Tablettenprüfvorrichtung mit einem Drehteller offenbart, bei welcher der Prüfkörper von einer drehbaren Richtplatte und einer Fernsehkamera zur Lagekontrolle auf dem Drehteller ausgerichtet wird.

Die Druckschrift DE 196 54 612 A1 offenbart eine Tablettenpresse, bei der eine Rinne zwischen der beweglichen Brechbacke und der feststehenden Messbacke die Prüfkörper ausrichtet.

Aufgabe der vorliegenden Erfindung ist es daher, eine Vorrichtung zur Überprüfung von Tabletten oder Oblongs bereitzustellen, die die Tablette oder die Oblongs vor ihrer Überprüfung einfach und schnell positioniert und eine problemlose Reinigbarkeit gewährleistet.

Diese Aufgabe wird durch die Merkmale des Patentanspruchs 1 gelöst. Weitere vorteilhafte Ausgestaltungen der Erfindung sind jeweils Gegenstand der Unteransprüche. Diese können in technologisch sinnvoller Weise miteinander kombiniert werden. Die Beschreibung, insbesondere im Zusammenhang mit der Zeichnung, charakterisiert und spezifiziert die Erfindung zusätzlich.[A1] Die Lösung der Aufgabe gelingt bei einer Vorrichtung gemäß Anspruch 1. Auch bei Prüfgeräten, in die der Prüfling manuell eingelegt werden muss, hilft die Positionierungsnut den Prüfling auszurichten. Die Nut ist vorzugsweise an die Form des Prüflings angepasst, sodass der Prüfling durch die Schwerkraft sich in der Nut selbst ausrichtet, indem er der die tiefste Lage einnimmt. In dieser Position verharrt er, bis er von der beweglichen Backe erfasst wird. Diese nimmt den Prüfling mit, wobei der Prüfling in der Nut in seiner Ausrichtung geführt wird, bis er auf die feste Backe trifft, um z.B. die Abmessung in der Bewegungsrichtung der Backe zu messen, sein Gewicht zu bestimmen oder den Prüfling einer Bruchprüfung zu unterziehen. [A2] Mit Vorteil können mehrere unterschiedlich profilierte Positionierungsnuten in der Stützfläche vorgesehen sein. Man erspart sich damit einen Stützflächenwechsel bei der Prüfung unterschiedlich geformter Prüflinge. Man legt dann jeweils nur den jeweiligen Prüfling in die dazu angepasste Positionierungsnut ein.

Dabei verändert sich der Berührungspunkt des Prüflings auf den Backen. Um dies zu vermeiden kann es vorteilhaft sein, wenn die Stützfläche in der die mindestens eine Positionierungsnut angeordnet ist, translatorisch quer zur Bewegungsrichtung der beweglichen Backe und/oder rotatorisch bewegbar ausgebildet ist.
[A3] Weiter ist es vorgesehen, dass die Stützfläche mit der mindestens einen Positionierungsnut als Drehteller ausgebildet ist. Durch diese Maßnahme gestaltet sich die Umschaltung der Positionierungsnuten einfacher. Es ergeben sich dadurch keine Endlagen, wie bei einer begrenzten translatorischen Bewegung. Bei einer rotatorischen Bewegung kann die Stützfläche immer um eine oder mehrere Rastpositionen weitergeschaltet werden.
[A4] Besonders zweckmäßig ist es, dass die mindestens eine Positionierungsnut in mindestens einem Quadranten des Drehtellers parallel zur Bewegungsrichtung der beweglichen Backe angeordnet ist und sich von einem Punkt auf einem Radialstrahl senkrecht zu diesem bis zum Umfang erstreckt.[A5] Dabei wird das Weiterschalten erheblich erleichtert, wenn der Drehteller einen Antrieb aufweist.
[A6] Der Drehteller weist eine vertikale Drehachse auf, die von der Längsachse der mindestens einen Positionierungsnut einen Abstand hat.[A7] Besonders vorteilhaft ist es, wenn eine Zuführeinrichtung für einen Prüfling zur Positionierungsnut, vorzugsweise in Form eines Transportsterns, oberhalb des Drehtellers vorgesehen ist, weil dann eine Seitenwand des Transportsterns den Prüfling selbsttätig in die Positionierungsnut bugsiert.[A8] Die Reproduzierbarkeit der Lagegenauigkeit der geschalteten Positionierungsnut wird vorteilhaft dadurch verbessert, dass der Drehteller eine Indexierung aufweist, die den Drehteller in einer Winkellage fixiert, in der der Radialstrahl, von dem die mindestens eine Positioniernut sich zum Umfang erstreckt, senkrecht zur Bewegungsrichtung der mindestens einen beweglichen Backe angeordnet ist.[A9] Wenn der Drehteller eine Öffnung als Auslass für getestete Tabletten aufweist, vereinfacht sich die Handhabung des Prüfgerätes und die Produktivität der Vorrichtung wird vorteilhaft gesteigert.
[A10] Demselben Zweck dient die Maßnahme, dass eine Reinigungsvorrichtung zur Entfernung von Prüflingen und/oder Bruchstücken aus der mindestens einen Positionierungsnut für den Drehteller vorgesehen ist.

Eine bevorzugte Ausführungsform der Erfindung wird beispielhaft an Hand einer Zeichnung erläutert. Die Figuren der Zeichnung zeigen im Einzelnen:
- Figur 1: eine perspektivische Ansicht auf den erfindungsgemäßen schematisch dargestellten Drehteller,
- Figur 2: eine Aufsicht auf den erfindungsgemäßen Drehteller schematisch ergänzt um weitere Vorrichtungsteile,
- Figur 3: eine Untersicht des Drehtellers gemäß Figur 1 und
- Figur 4: eine Seitenansicht des Drehtellers gemäß Figur 1.

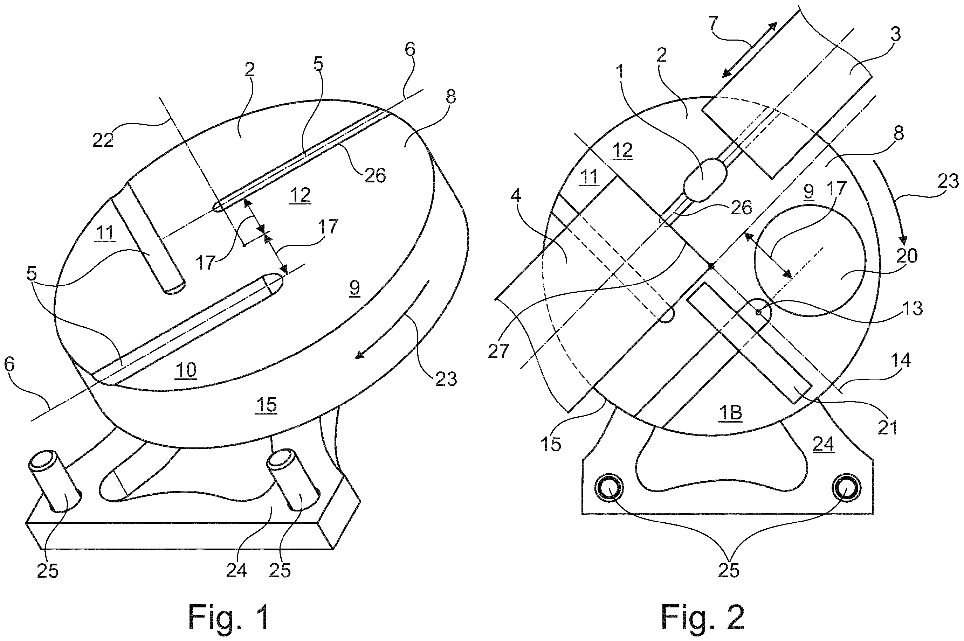

Figur 1 zeigt einen erfindungsgemäßen Drehteller 8 in perspektivischer Ansicht. In die obere Stützfläche 2 sind insgesamt 3 Positionierungsnuten 5 eingetieft. Ein Quadrant weist keine Positionierungsnut 5 auf, während die übrigen Quadranten jeweils eine Positionierungsnut besitzen. Die Positionierungsnuten sind dabei unterschiedlich breit und tief ausgeführt, damit sie unterschiedliche Größen von Prüflingen aufnehmen und ausrichten können.

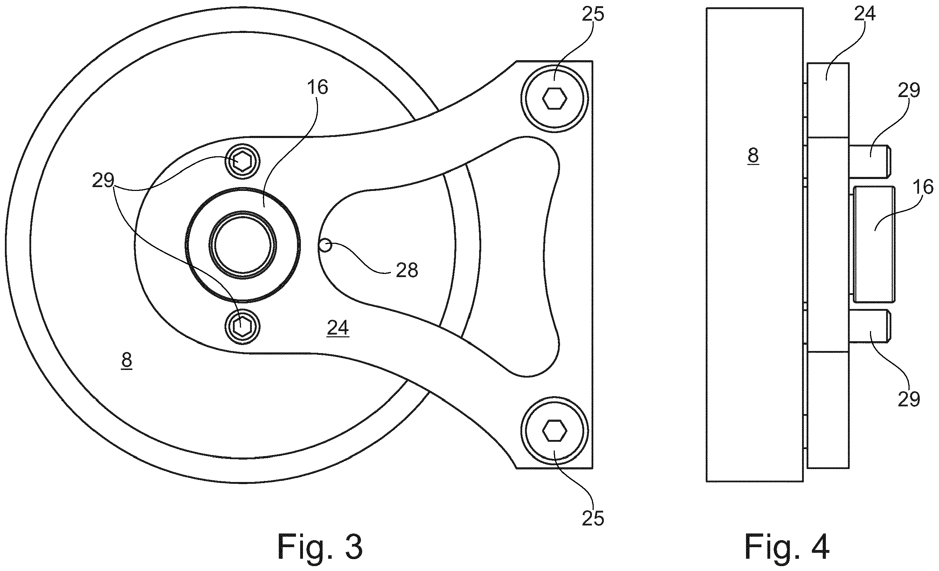

Der Drehteller 8 dreht sich um die Achse 22 und weist insgesamt vier Rastpositionen auf, die den einzelnen Quadranten 9, 10, 11 und 12 zugeordnet sind. Von einer Rastposition zur nächsten Rastposition bewegt sich der Drehteller 8 mittels Antrieb 16 in die Drehrichtung 23. Als Drehmomentstütze des Antriebs 16 (Figur 3, Figur 4) dient dabei eine Halterung 24 die mittels der Gewindeschrauben 25 in dem jeweiligen Prüfgerät befestigt werden können.

In Figur 2 sind weitere Teile der Vorrichtung schematisch in einer Aufsicht dargestellt. Die eigentliche Prüfvorrichtung mit beweglicher Backe 3 und feststehender Backe 4 überdeckt im Falle der Figur 2 die Quadranten 11 und 12. Zwischen diesen Backen 3, 4 ist ein Prüfling 1 dargestellt, der in der Positionierungsnut 26 ausgerichtet liegt und darauf wartet, dass ihn die bewegliche Backe 3 durch eine Bewegung in Richtung 7, die parallel zur Längsachse 6 der Positionierungsnut 5 verläuft, berührt und gegen die Anschlagfläche 27 der Festbacke presst.

Die Längsachsen 6 der Positionierungsnuten 5 weisen einen Abstand 17 zur Drehachse 22 des Drehtellers 8 auf.

In dem von einer Positionierungsnut 5 freien Quadrant 9 ist eine Öffnung 20 vorgesehen, durch die Bruchstücke des Prüflings hindurchfallen können, sobald sie von der Reinigungsvorrichtung 21 erfasst und zurückgehalten werden, bis sich die Öffnung 20 unter der Reinigungsvorrichtung vorbei bewegt. Beim weiteren Drehen des Drehtellers 8 in Richtung 23 unterfährt nämlich die Öffnung 20 die Reinigungsvorrichtung, beispielsweise eine Bürste, so dass die von der Bürste zurückgehaltenen Bruchstücke des Prüflings dabei durch die Öffnung 20 fallen.

Die feststehende Backe 4 und die Reinigungsvorrichtung 21 sowie die Halterung 24 sind stationär im Prüfgerät befestigt, während sich der Drehteller 8 in Drehrichtung 23 intervallweise rotierend bewegt und die bewegliche Backe 3 translatorisch in Richtung 7 verfahrbar ist.

Auch wenn ständig dieselben Prüflinge 1 getestet werden, kann der Drehteller 8 zwischen zwei Prüfungen eine Drehung in Richtung 23 um 360° vollziehen, damit der Prüfling oder die Bruchstücke des Prüflings 1 durch die Öffnung 20 fallen können und sich anschließend dieselbe Positionierungsnut 26 wieder zwischen den Backen 3 und 4 befindet.

In Figur 3 ist eine Sicht von unten auf den erfindungsgemäßen Drehteller 8 dargestellt, während Figur 4 eine Seitenansicht zeigt. Der Motor 16 ist mittels zweier gegenüberliegende Schrauben 29 drehfest mit der Halterung 24 verbunden. Außerdem ist eine Indexierung 28 sichtbar. Diese ist als Bohrung ausgeführt, in die ein in diesen Darstellungen nicht sichtbarer Indexstift der Rastpositionen eingreift. Es gehört zum fachmännischen Können eine entsprechende Indexierung vorzusehen.

Auf diese Weise ist eine Prüfvorrichtung geschaffen worden, die auf einfache und schnelle Weise an unterschiedliche Prüflinge angepasst werden kann.

### Bezugszeichenliste

- 1: Prüfling
- 2: Stützfläche
- 3: beweglichen Backe
- 4: feststehenden Backe
- 5: Positionierungsnut
- 6: Längsachse
- 7: Bewegungsrichtung
- 8: Drehteller
- 9: Quadrant
- 10: Quadrant
- 11: Quadrant
- 12: Quadrant
- 13: Punkt
- 14: Radialstrahl
- 15: Umfang
- 16: Antrieb
- 17: Abstand
- 18: Zuführeinrichtung (nicht dargestellt)
- 19: Transportstern (nicht dargestellt)
- 20: Öffnung
- 21: Reinigungsvorrichtung
- 22: Drehachse
- 23: Drehrichtung
- 24: Halterung
- 25: Gewindeschraube
- 26: Positionierungsnut
- 27: Anschlagfläche
- 28: Indexierung
- 29: Schraube

## Patentansprüche

1. Vorrichtung zur Prüfung von Tabletten oder Oblongs als Prüfling (1) mit einer Stützfläche (2) für eine zu prüfende Tablette zwischen zwei beabstandeten Backen, wobei die eine Backe (3) beweglich und die andere Backe (4) feststehend ist, wobei
die Stützfläche (2) zwischen den Backen (3, 4) mindestens eine Positionierungsnut (5) aufweist, deren Längsachse (6) der Bewegungsrichtung (7) der mindestens einen beweglichen Backe (3) entsprechend angeordnet ist, **dadurch gekennzeichnet, dass** die Stützfläche (2) mit der mindestens einen Positionierungsnut (5) als Drehteller (8) ausgebildet ist, wobei der Drehteller (8) eine vertikale Drehachse (22) aufweist, die von der Längsachse (6) der mindestens einen Positionierungsnut einen Abstand (17) aufweist, wobei die mindestens eine Positionierungsnut (5) in mindestens einem Quadranten (9, 10, 11, 12) des Drehtellers (8) parallel zur Bewegungsrichtung (7) der beweglichen Backe (3) angeordnet ist und sich von einem Punkt (13) auf einem Radialstrahl (14) senkrecht zu diesem bis zum Umfang (15) des Drehtellers (8) erstreckt.

2. Vorrichtung zur Prüfung von Tabletten nach Anspruch 1,
**dadurch gekennzeichnet, dass** die Stützfläche (2) in der die mindestens eine Positionierungsnut (5) angeordnet ist, translatorisch quer zur Bewegungsrichtung (7) der beweglichen Backe (3) und/oder rotatorisch beweglich ausgebildet ist.

3. Vorrichtung zur Prüfung von Tabletten nach Anspruch 1,
**dadurch gekennzeichnet, dass** der Drehteller (8) einen Antrieb (16) aufweist.

4. Vorrichtung zur Prüfung von Tabletten nach einem der vorhergehenden Ansprüche,
**dadurch gekennzeichnet, dass** eine Zuführeinrichtung (18) für einen Prüfling (1) zur Positionierungsnut (5), vorzugsweise in Form eines Transportsterns (19), oberhalb des Drehtellers (8) vorgesehen ist.

5. Vorrichtung zur Prüfung von Tabletten nach einem der vorhergehenden Ansprüche,
**dadurch gekennzeichnet, dass** der Drehteller (8) eine Indexierung (28) aufweist, die den Drehteller (8) in einer Winkellage fixiert, in der der Radialstrahl (14) von dem die mindestens eine Positioniernut (5) sich zum Umfang (15) erstreckt senkrecht zur Bewegungsrichtung (7) der mindestens einen beweglichen Backe (3) angeordnet ist.

6. Vorrichtung zur Prüfung von Tabletten nach einem der vorhergehenden Ansprüche,
**dadurch gekennzeichnet, dass** der Drehteller (8) eine Öffnung (20) als Auslass für getestete Tabletten (1) aufweist.

7. Vorrichtung zur Prüfung von Tabletten nach einem der vorhergehenden Ansprüche,
**dadurch gekennzeichnet, dass** eine Reinigungsvorrichtung (21) zur Entfernung von Staub und/oder Bruchstücken aus der mindestens einen Positionierungsnut (5) für den Drehteller (8) vorgesehen ist.

## Claims

1. Device for testing tablets or oblongs as samples (1), with a supporting surface (2) for a tablet to be tested between two jaws (4) spaced apart, wherein the one jaw (3) is movable and the other jaw (4) is stationary, wherein
the supporting surface (2) comprises between the jaws (3, 4) at least one positioning groove (5) whose longitudinal axis (6) is located corresponding to the direction of movement (7) of the at least one movable jaw (3),
**characterized in that** the supporting surface (2) with the at least one positioning groove (5) is implemented as a rotary plate (8), the rotary plate (8) having a vertical rotational axis (22) spaced apart from the longitudinal axis (6) of the at least one positioning groove (5) at a distance (17), wherein the at least one positioning groove (5) is disposed in at least one quadrant (9, 10, 11, 12) of the rotary plate (8) parallel to the direction of movement (7) of the movable jaw (3) and extends from a point (13) on a radius (14) perpendicularly to the same up to the periphery (15) of the rotary plate (8).

2. Device for testing tablets as in claim 1,
**characterized in that** the supporting surface (2) in which the at least one positioning groove (5) is disposed is implemented such that it is translationally transverse to the direction of movement (7) of the movable jaw (3) and/or rotationally movable.

3. Device for testing tablets as in claim 1,
**characterized in that** the rotary plate (8) comprises a drive (16).

4. Device for testing tablets as in one of the preceding claims,
**characterized in that** a feeding system (18), preferably in the form of a transporting starwheel (19), for a sample (1) to the positioning groove (5) is provided above the rotary plate (8).

5. Device for testing tablets as in one of the preceding claims,
**characterized in that** the rotary plate (8) includes an indexation (28) which fixes the rotary plate (8) in an angular position in which the radius (14), from which extends the at least one positioning groove (5) to the periphery (15), is located perpendicularly to the direction of movement (7) of the at least one movable jaw (3).

6. Device for testing tablets as in one of the preceding claims,
**characterized in that** the rotary plate (8) comprises an opening (20) as an outlet for tested tablets (1).

7. Device for testing tablets as in one of the preceding claims,
**characterized in that** a cleaning means (21) for the removal of dust and/or fragments from the at least one positioning groove (5) is provided for the rotary plate (8).

## Revendications

1. Dispositif de vérification de comprimés ou de gélules en tant qu'échantillon (1) comportant une surface de support (2) destinée à un comprimé à vérifier entre deux mâchoires mutuellement espacées, dans lequel l'une des mâchoires (3) est mobile et l'autre mâchoire (4) est stationnaire, dans lequel
la surface de support (2) comporte, entre les mâchoires (3, 4), au moins une fente de positionnement (5) dont l'axe longitudinal (6) est disposé de manière à correspondre à la direction de mouvement (7) de l'au moins une mâchoire mobile (3), **caractérisé en ce que** la surface de support (2) comportant l'au moins une fente de positionnement (5) est réalisée sous la forme d'un plateau tournant (8), dans lequel le plateau tournant (8) comporte un axe de rotation vertical (22) qui présente un espacement (17) par rapport à l'axe longitudinal (6) de l'au moins une fente de positionnement, dans lequel l'au moins une fente de positionnement (5) est disposée dans au moins un quadrant (9, 10, 11, 12) du plateau tournant (8) parallèlement à la direction de mouvement (7) de la mâchoire mobile (3) et s'étend depuis un point (13) situé sur un rayon (14) perpendiculaire à celui-ci jusqu'à la circonférence (15) du plateau tournant (8).

2. Dispositif de vérification de comprimés selon la revendication 1,
**caractérisé en ce que** la surface de support (2) dans laquelle est disposée l'au moins une fente de positionnement (5) est réalisée de manière à être mobile en translation transversalement à la direction de mouvement (7) de la mâchoire mobile (3) et/ou en rotation.

3. Dispositif de vérification de comprimés selon la revendication 1,
**caractérisé en ce que** le plateau tournant (8) comporte un dispositif d'entraînement (16).

4. Dispositif de vérification de comprimés selon l'une quelconque des revendications précédentes,
**caractérisé en ce qu'il** est prévu au-dessus du plateau tournant (8) un dispositif d'acheminement (18) d'un échantillon (1) vers la fente de positionnement (5), de préférence sous la forme d'un dispositif de transport en étoile (19).

5. Dispositif de vérification de comprimés selon l'une quelconque des revendications précédentes,
**caractérisé en ce que** le plateau tournant (8) présente un système d'indexation (28) qui fixe le plateau tournant (8) à une position angulaire à laquelle le rayon (14) par rapport auquel l'au moins une fente de positionnement (5) s'étend vers la circonférence (15) est disposé perpendiculairement à la direction de mouvement (7) de l'au moins une mâchoire mobile (3).

6. Dispositif de vérification de comprimés selon l'une quelconque des revendications précédentes,
**caractérisé en ce que** le plateau tournant (8) comporte une ouverture (20) servant d'orifice de sortie pour les comprimés testés (1).

7. Dispositif de vérification de comprimés selon l'une quelconque des revendications précédentes,
**caractérisé en ce qu'il** est prévu un dispositif de nettoyage (21) destiné à éliminer la salissure et/ou des fragments provenant de l'au moins une fente de positionnement (5) pour le plateau tournant (8).
